# EUROPÄISCHE PATENTSCHRIFT

(11) **EP 0 904 348 B1**
(45) Veröffentlichungstag und Bekanntmachung des Hinweises auf die Patenterteilung: **24.11.2004**
(21) Anmeldenummer: 97927092.3
(22) Anmeldetag: 30.05.1997
(51) Int. Cl.: C12N 1/20, C12N 9/80, C12P 13/00, C12P 13/02, C12P 41/00, C07C 233/23, C12R 1/01

(54) **VERFAHREN ZUR HERSTELLUNG VON AMINOALKOHOLEN UND DERIVATEN DAVON**
PROCESS FOR THE PREPARATION OF AMINO ALCOHOLS AND DERIVATIVES THEREOF
PROCEDE DE PRODUCTION D'AMINOALCOOLS ET DE LEURS DERIVES

(30) Priorität: 30.05.1996 CH 135996; 10.02.1997 CH 28297; 18.04.1997 CH 90897
(43) Veröffentlichungstag der Anmeldung: 31.03.1999
(73) Patentinhaber: Lonza AG, 3930 Visp (CH)
(72) Erfinder: BERNEGGER-EGLI, Christine, CH-3985 Münster (CH); BIRCH, Olwen, M., CH-3930 Visp (CH); BOSSARD, Pierre, CH-1213 Onex (CH); BRIEDEN, Walter, CH-3902 Glis (CH); BRUX, Frank, CH-3942 Raron (CH); BURGDORF, Knut, CH-3911 Ried-Brig (CH); DUC, Laurent, CH-3971 Chermignon (CH); ETTER, Kay-Sarah, CH-3945 Niedergampel (CH); GUGGISBERG, Ives, CH-3960 Sierre (CH); SAUTER, Martin, CH-3930 Visp (CH); URBAN, Eva, Maria, CH-3930 Visp (CH)
(74) Vertreter: Winter, Brandl & Partner
(86) Internationale Anmeldenummer: PCT/EP1997/002838
(87) Internationale Veröffentlichungsnummer: WO 1997/045529

(56) Entgegenhaltungen:
- WO-A-91/01318
- WO-A-91/01319
- WO-A-91/15447
- WO-A-93/17020
- CAMPBELL, JEFFREY A. ET AL: "Chirospecific Syntheses of Precursors of Cyclopentane and Cyclopentene Carbocyclic Nucleosides by [3 + 3]-Coupling and Transannular Alkylation" J. ORG. CHEM. (1995), 60(14), 4602-16 CODEN: JOCEAH;ISSN: 0022-3263, 1995, XP002041314 in der Anmeldung erwähnt
- PARK K H ET AL: "Enantioselective synthesis of (1R,4S)-1- amino -4-( hydroxymethyl )-2- cyclopentene, a precursor for carbocyclic nucleoside synthesis." JOURNAL OF ORGANIC CHEMISTRY 59 (2). 1994. 394-399. ISSN: 0022-3263, XP002041315 in der Anmeldung erwähnt
- S.J.C. TAYLOR ET AL.: "Development of the Biocatalytic resolution of 2-azabicyclo[2.2.1]hept-5-en-3-one as an entry to single enantiomer carbocyclic nucleosides." TETRAHEDRON : ASYMMETRY, Bd. 4, Nr. 6, 1993, Seiten 1117-1128, XP002041316 in der Anmeldung erwähnt
- L.E. MART NEZ ET AL.: "Highly efficient and enantioselective synthesis of carocyclic nucleoside analogs using selective early transition metal catalysis." J. ORG. CHEM, Bd. 61, 1996, Seiten 7963-7966, XP002041317 in der Anmeldung erwähnt
- M.H. NORMAN ET AL.: "Novel synthesis of (+/-)-cis-4-amino-2-cyclopentene-1-methano l, a key intermediate in the preparation of carbocyclic 2',3'-didehydro-2',3'-dideoxy nucleosides." SYNTHETIC COMMUNICATIONS, Bd. 22, Nr. 22, 1992, Seiten 3197-3204, XP002041482

## Beschreibung

Die Erfindung betrifft ein neues Verfahren zur Herstellung von (1R,4S)- oder (1S,4R)-1-Amino-4-(hydroxymethyl)-2-cyclopenten der Formeln 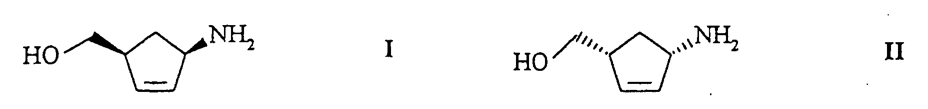 und / oder von (1S,4R)- oder (1R,4S)-Aminoalkoholderivaten der allgemeinen Formeln 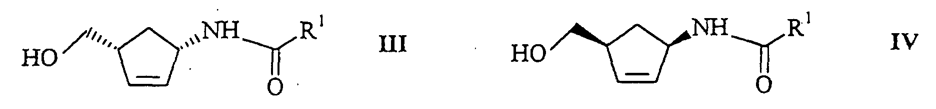 sowie neue Mikroorganismen, die befähigt sind, ein Cyclopentenderivat der allgemeinen Formel 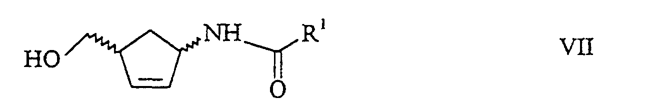 als einzige Stickstoffquelle, als einzige Kohlenstoffquelle oder als einzige Kohlenstoff- und Sticfkstoffquelle zu verwerten.

Die Erfindung betrifft ferner Enzyme mit N-Acetylaminoalkoholhydrolase-Aktivität, die aus diesen Mikroorganismen erhältlich sind.

(1R,4S)-1-Amino-4-(hydroxymethyl)-2-cyclopenten der Formel I ist ein wichtiges Zwischenprodukt zur Herstellung von carbocyclischen Nukleosiden wie z. B. Carbovir® (Campbell et al., J. Org. Chem. 1995, 60, 4602 - 4616).

Verfahren zur Herstellung von (1R,4S)-1-Amino-4-(hydroxymethyl)-2-cyclopenten sind von Campbell et al. (ibid) und von Park K. H. & Rapoport H. (J. Org. Chem. 1994, 59, 394 - 399) beschrieben.

Bei diesen Verfahren dient als Edukt entweder D-Glucon-δ-lacton oder D-Serin, wobei ca. 15 Synthesestufen bis zur Bildung von (1R,4S)-N-tert.Butoxycarbonyl-4-hydroxymethyl-2-cyclopenten, welches dann zum (1R,4S)-1-Amino-4-(hydroxymethyl)-2-cyclopenten entscheitzt wird, notwendig sind.
Diese beiden Verfahren sind kostspielig, umständlich und grosstechnisch nicht gangbar.

Die WO 93/17020 beschreibt ein Verfahren zur Herstellung von (1R,4S)-1-Amino-4-(hydroxymethyl)-2-cyclopenten, wobei (1R,4S)-4-Amino-2-cyclopenten-1-carbonsäure mit Lithiumaluminiumhydrid zum gewünschten Produkt reduziert wird.

Nachteilig bei diesem Verfahren ist, dass auch die Doppelbindung des Cyclopentenringes reduziert wird, die schlechte Handhabbarkeit von Lithiumaluminiumhydrid und dass es zu kostspielig ist.

Taylor, S. J. et al. (Tetrahetron: Asymmetry Vol. 4, No. 6, 1993, 1117 - 1128) beschreiben ein Verfahren zur Herstellung von (1R,4S)-1-Amino-4-(hydroxymethyl)-2-cyclopenten ausgehend von (±)-2-Azabicyclo[2.2.1]hept-5-en-3-on als Edukt. Dabei wird das Edukt mittels Mikroorganismen der Spezies Pseudomonas solanacearum oder Pseudomonas fluorescens in (1R,4S)-2-Azabicyclo[2.2.1]hept-5-en-3-on überführt, welches dann mit Di-tert.butyldicarbonat in das (1R,4S)-N-tert.-Butoxycarbonyl-2-azabicyclo[2.2.1]hept-5-en-3-on umgesetzt wird, das mit Natriumborhydrid und Trifluoressigsäure zum gewünschten Produkt reduziert wird.
Dieses Verfahren ist viel zu kostspielig.

Desweiteren beschreiben Martinez et al. (J. Org. Chem. 1996, 61, 7963 - 7966) eine 10stufige Synthese von (1R,4S)-1-Amino-4-(hydroxymethyl)-2-cyclopenten ausgehend von Dialkylmalonsäurediethylester. Auch dieses Verfahren hat den Nachteil, dass es umständlich und grosstechnisch nicht gangbar ist.

Aufgabe der vorliegenden Erfindung war, ein einfaches Verfahren zur Herstellung von (1R,4S)-1-Amino-4-(hydroxymethyl)-2-cyclopenten zur Verfügung zu stellen.

Diese Aufgabe wird mit dem erfindungsgemässen Verfahren nach Anspruch 6 gelöst. Die Mikroorganismen nach Anspruch 1 sowie die Enzyme nach Anspruch 2 sind ebenfalls Gegenstand der Erfindung.

Mikroorganismen zur Verwendung in dem erfindungsgemäßen Verfahren können aus Bodenproben, Schlamm oder Abwasser unter Zuhilfenahme üblicher mikrobiologischer Techniken isoliert werden.

Erfindungsgemäss erfolgt die Isolation der Mikroorganismen derart, dass man diese in einem Nährmedium, enthaltend ein oder mehrere Cyclopentenderivate der allgemeinen Formel 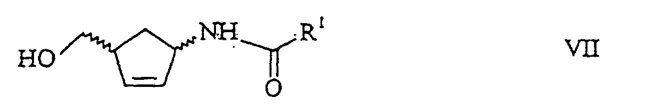 worin R¹ C₁-C₄-Alkyl, C₁-C₄-Alkoxy, Aryl oder Aryloxy bedeutet,
- als einzige Kohlenstoff- und Stickstoffquelle
- als einzige Stickstoffquelle mit einer geeigneten Kohlenstoffquelle oder
- als einzige Kohlenstoffquelle mit einer geeigneten Stickstoffquelle,
auf übliche Weise züchtet.

Als C₁-C₄-Alkyl kann beispielsweise Methyl, Ethyl, Propyl, Isopropyl oder Butyl verwendet werden.
Als C₁-C₄-Alkoxy kann beispielsweise Methoxy, Ethoxy, Propoxy, Isopropoxy, Butoxy oder tert.-Butoxy verwendet werden.
Als Aryl kann beispielsweise Phenyl oder Benzyl verwendet werden. Vorzugsweise wird Benzyl verwendet.
Als Aryloxy kann beispielsweise Benzyloxy oder Phenoxy verwendet werden.
Demzufolge sind als Cyclopentenderivat der allgemeinen Formel VII beispielsweise geeignet: N-Acetyl-1-amino-4-hydroxymethyl-2-cyclopenten, N-butyryl-1-amino-4-hydroxymethyl-2-cyclopenten oder N-phenylacetyl-1-amino-4-hydroxymethyl-2-cyclopenten.

Zweckmässig werden aus der durch Züchtung erhaltenen Kultur die Mikroorganismen selektioniert, die das (1R,4S)-Isomere des Cyclopentenderivats der Formel VII als einzige Stickstoffquelle, als einzige Kohlenstoffquelle oder als einzige Kohlenstoff- und Stickstoffquelle verwerten.

Als geeignete Stickstoffquelle können die Mikroorganismen beispielsweise Ammonium, Nitrate, Aminosäuren oder Harnstoffe als Wachstumssubstrat nützen.
Als geeignete Kohlenstoffquelle können die Mikroorganismen beispielsweise Zucker, Zuckeralkohole, C₂-C₄-Carbonsäuren oder Aminosäuren als Wachstumssubstrat nützen. Als Zucker können Hexosen wie Glucose oder Pentosen verwendet werden. Als Zuckeralkohol kann beispielsweise Glycerin Verwendung finden. Als C₂-C₄-Carbonsäuren können beispielsweise Essigsäure oder Propionsäure verwendet werden. Als Aminosäuren können beispielsweise Leucin, Alanin, Asparagin verwendet werden.

Als Selektions- und Anzuchtmedium können die in der Fachwelt üblichen verwendet werden, wie beispielsweise das in Tabelle 1 beschriebene oder ein Vollmedium (Medium enthaltend Hefe-Extrakt), vorzugsweise wird das in Tabelle 1 beschriebene verwendet.

Während der Anzucht und Selektion werden zweckmässig die wirksamen Enzyme der Mikroorganismen induziert. Als Enzyminduktor können die Cyclopentenderivate der allgemeinen Formel VII verwendet werden.
Üblicherweise erfolgt die Anzucht und Selektion bei einer Temperatur von 20 °C bis 40 °C, vorzugsweise von 30 °C bis 38 °C und bei einem pH zwischen 5,5 und 8,0, vorzugsweise zwischen 6,8 und 7,8.

Bevorzugte Mikroorganismen sind solche der Gattung Rhodococcus, Gordona, Arthrobacter, Alcaligenes, Agrobacterium / Rhizobium, Bacillus, Pseudomonas oder Alcaligenes / Bordetella insbesondere der Spezies Rhodococcus erythropolis CB 101 (DSM 10686), Alcaligenes Bordetella FB 188 (DSM 111.72), Arthrobacter sp. HSZ 5 (DSM 10328), Rhodococcus sp. FB 387 (DSM 11291), Alcaligenes xylosoxydans ssp. denitrificans HSZ 17 (DSM 10329) oder Gordona sp. CB 100 (DSM 1068.7) sowie deren funktionell aequivalente Varianten und Mutanten. Die Mikroorganismen DSM 10686 und 10687 wurden am 20.05.1996, die Mikroorganismen DSM 10328 und DSM 10329 am 6.11.1995, die Mikroorganismen DSM 11291 am 8.10.1996 und die Mikroorganismen DSM 11172 am 20.09.1996 bei der Deutschen Sammlung von Mikroorganismen und Zellkulturen GmbH (DSMZ), Mascherorderweg 1b, D-38124 Braunschweig, gemäss Budapester Vertrag hinterlegt.

Unter "funktionell äquivalenten Varianten und Mutanten" werden Mikroorganismen verstanden, die im wesentlichen dieselben Eigenschaften und Funktionen wie die Ursprungsmikrooganismen besitzen. Derartige Varianten und Mutanten können zufällig, z. B. durch UV-Bestrahlung, gebildet werden.

### Taxonomische Beschreibung von Alcaligenes / Bordetella FB 188 (DSM 11172)

| | |
|---|---|
| Zellform | Stäbchen |
| Breite µm | 0,5 - 0,6 |
| Länge µm | 1,0 - 2,5 |
| Beweglichkeit | + |
| Begeisselung | peritrich |
| Gram-Reaktion | - |
| Lyse durch 3% KOH | + |
| Aminopeptidase (Cerny) | + |
| Sporen | - |
| Oxidase | + |
| Catalase | + |
| ADH (Alkoholdehydrogenase) | - |
| NO₂ aus NO₃ | - |
| Denitrifikation | - |
| Urease | - |
| Hydrolyse von Gelatine | - |
| Säure aus (OF-Test): | |
| Glucose | - |
| Fructose | - |
| Arabinose | - |
| Adipat | + |
| Caprat | + |
| Citrat | + |
| Malat | + |
| Mannitol | - |

### Taxonomische Beschreibung von Rhodococcus erythropolis CB 101 (DSM 106 86)

1. Morphologie und Farbe der Kolonien: Kurze verzweigte Hyphen, die im Alter in Stäbchen und Kokken zerfallen, Kolonien glänzend zum Teil zerfliessend, beige mit rosa Ton, RAL 1001;
2. Diagnostizierte Aminosäure des Peptidglycans: meso-Diaminopimelinsäure;
3. Mycolsäuren: Rhodococcus-Mycolsäuren; Die Bestimmung der Mycolsäure-Kettenlänge (C₃₂ - C₄₄) und der Vergleich der Daten mit den Einträgen in der DSM-Mycolsäure-Datenbank ergab eine sehr hohe Ähnlichkeit zu den Mustern der Rhodococcus erythropolis Stämmen (Similarität, 0,588).
4. Fettsäuremuster: Unverzweigte, gesättigte und ungesättigte Fettsäuren plus Tuberculostearinsäure.
5. Bei der Partial-Sequenzierung der 16S rDNA des Stammes wurde eine hohe Übereinstimmung (100%) mit den Sequenzen der spezifischen Regionen von Rhodococcus erythropolis gefunden.

Das Identifizierungsergebnis ist eindeutig, da drei voneinander unabhängige Methoden (Mycolsäuren, Fettsäuren, 16S rDNA) den Stamm der Spezies Rhodococcus erythropolis zugeordnet haben.

### Taxonomische Beschreibung von Gordona sp. CB 100 (DSM 10687)

1. Morphologie und Farbe der Kolonien: Kurze verzweigte Hyphen, die im Alter in Stäbchen und Kokken zerfalllen, Kolonien hellorange, (RAL 2008);
2. Diagnostizierte Aminosäure des Peptidoglycans: meso-Diaminopimelinsäure;
3. Menachinonmuster: MK-9 (H₂) 100%;
4. Mycolsäuren: Gordona-Mycolsäuren; Die Bestimmung der Mycolsäure-Kettenlänge (C₅₀ - C₆₀) erfolgte mit Hilfe von Hochtemperatur-Gaschromatographie. Dieses Muster entspricht dem Muster, wie es bei Vertretern der Gattung Gordona gefunden wird.
5. Fettsäuremuster: Unverzweigte, gesättigte und ungesättigte Fettsäuren plus Tuberculostearinsäure.
6. Bei der Partial-Sequenzierung der 16S rDNA des Stammes konnte nur eine relativ niedrige Übereinstimmung 98,8% mit den Sequenzen der spezifischen Regionen von Gordona rubropertincta gefunden werden.
   Aufgrund der vorliegenden Ergebnisse (Menachinone, Mycolsäuren, Fettsäuren 16S rDNA) kann das Isolat zwar eindeutig der Gattung Gordona zugeordnet werden, eine Zuordnung zu einer bekannten Gordona Spezies ist auf Grund dieser Ergebnisse nicht möglich. Es ist daher anzunehmen, dass es sich bei dem Stamm DSM 10687 um eine neue bisher nicht beschriebene Spezies der Gattung Gordona handelt.

### Taxonomische Beschreibung von Alcaligenes xylosoxydans ssp. denitrificans HSZ 17 (DSM 10329)

Eigenschaften des Stammes

| | |
|---|---|
| Zellform | Stäbchen |
| Breite µm | 0,5-0,6 |
| Länge µm | 1,5-3,0 |
| | |
| Beweglichkeit | + |
| | |
| Begeisselung | peritrich |
| | |
| Gram-Reaktion | - |
| Lyse durch 3% KOH | + |
| Aminopeptidase (Cerny) | + |
| Sporen | - |
| | |
| Oxidase | + |
| | |
| Catalase | + |
| | |
| Wachstum anaerob | - |
| | |
| ADH (Alkoholdehydrogenase) | + |
| | |
| NO₂ aus NO₃ | + |
| | |
| Denitrifikation | + |
| | |
| Urease | - |
| | |
| Hydrolyse von | |
| Gelatine | - |
| Tween 80 | - |
| | |
| Säure aus (OF-Test): | |
| Glucose aerob | - |
| Xylose 80 | - |
| | |
| Substratverwertung | |
| Glucose | - |
| Fructose | - |
| Arabinose | - |
| Citrat | + |
| Malat | + |
| Mannitol | - |

### Taxonomische Beschreibung von Arthrobacter sp. HSZ5 (DSM 10328)

| | |
|---|---|
| Charakterisierung: | Gram-positive unregelmässige Stäbchen mit einem ausgeprägten Stäbchen-Kokken Wachstumszyklus; strikt aerob; keine Säure- oder Gasbildung aus Glucose. |
| | |
| Beweglichkeit | - |
| Sporen | - |
| Katalase | + |

*meso*-Diaminopimelinsäure in der Zellwand: nein
Peptidoglycan-Typ: A3α, L-Lys-L-Ser-L-Thr-L-Ala
- 16S rDNA Sequenz-Ähnlichkeit:: Sequenzierung des Bereichs mit der grössten Variabilität ergab als höchste Werte 98,2% mit Arthrobacter pascens, A. ramosus und A. oxydans

### Taxonomische Beschreibung von Rhodococcus sp. FB 387 (DSM 11291)

1. Morphologie und Farbe der Kolonien: Kurze verzweigte Hyphen, die im Alter in Stäbchen und Kokken zerfallen, Kolonien stumpf, hell rot-orange RAL 2008;
2. Diagnostizierte Aminosäure des Peptidoglycans: meso-Diaminopimelinsäure;
3. Mycolsäuren: Rhodococcus-Mycolsäuren;
   Die Bestimmung der Mycolsäure-Kettenlänge (C₃₂-C₄₄) und der Vergleich der Daten mit den Einträgen in der DSMZ-Mycolsäuredatenbank ergab nur eine sehr geringe Ähnlichkeit zu den Mustern von Rodococcus ruber Stämmen (Similarität, 0,019). Dieser Korrelationsfaktor ist zu gering, um zur Spezies Identifizierung herangezogen werden zu können.
4. Fettsäuremuster: Unverzweigte, gesättigte und ungesättigte Fettsäuren plus Tuberculostearinsäure.
   Dieses Fettsäuremuster ist diagnostisch für alle Vertreter der Gattung Rhodococcus und ihre nahen Verwandten wie Mycobacterium, Nocardia und Gordona. Es wurde versucht, unter Einbeziehung der qualitativen und quantitativen Unterschiede im Fettsäuremuster eine Differenzierung zur Spezies-Ebene durchzuführen. Mit Hilfe von numerischen Methoden wurde das Fettsäure-Muster von Rodococcus sp. FB 387 mit den Einträgen der Datenbank verglichen. Auch mit dieser Methode konnte Rodococcus sp. FB 387 auf Grund der geringen Similarität (0,063) keiner beschriebenen Rhodococcus-Spezies zugeordnet werden.
5. Bei der Partial-Sequenzierung der 16S rDNA des Stammes wurde 96-818 mit einer Korrelation von 97,9% Rhodococcus opacus zugeordnet. Diese Sequenzübereinstimmung liegt weit unter der von 99,5% wie sie für eine eindeutige Spezies-Zuordnung in diesem Taxon verlangt wird.

Auf Grund der vorliegenden Ergebnisse kann man davon ausgehen, dass es sich bei dem Stamm Rhodococcus sp. FB 387 um eine neue bisher nicht beschriebene Rhodococcus-Spezies handelt.

Enzyme zur Verwendung in dem erfindungsgemäßen Verfahren, die N-Acetylaminoalkoholhydrolasen, die befähigt sind, Cyclopentenderivate der obigen Formel VII zu hydrolysieren, können zum Beispiel durch fachmännisch übliches Aufschliessen der beschriebenen Mikroorganismenzellen gewonnen werden. Hierzu kann beispielsweise die Ultraschall- oder French-Press-Methode verwendet werden. Beispielsweise sind diese Enzyme aus den Mikroorganismen Rhodococcus erythropolis CB 101 (DSM 10686) erhältlich. Solche Enzyme sind bevorzugt gekennzeichnet durch:
a) eine N-terminale Aminosäuresequenz von Thr-Glu-Gln-Asn-Leu-His-Trp-Leu-Ser-Ala-Thr-Glu-Met-Ala-Ala-Ser-Val-Ala-Ser-Asn;
b) ein Molekulargewicht von 50 kD, bestimmt durch SDS-PAGE;
c) ein pH-Optimum von pH 7,0 ± 1,0;
d) ein Temperatur-Optimum zwischen 25 °C und 30 °C bei einem pH-Wert von 7,0; und
e) einen K_{M}-Wert für das Substrat N-acetyl-1-amino-4-hydroxymethyl-2-cyclopenten von 22,5 mM ± 7,5 mM (30 °C, 100 mM Phosphat Puffer, pH 7,0).

Enzyme, wie sie aus den erfindungsgemäßen Mikroorganismen, beispielsweise Rhodococcus erythropolis CB 101 (DSM 10686), erhältlich sind, hydrolysieren beispielsweise insbesondere N-acetyl-1-amino-4-hydroxymethyl-2-cyclopenten, N-butyryl-1-amino-4-hydroxymethyl-2-cyclopenten, N-propionyl-1-amino-4-hydroxymethyl-2-cyclopenten, und N-isobutyryl-1-amino-4-hydroxymethyl-2-cyclopenten.

Das erfindungsgemässe Verfahren zur Herstellung von (1R,4S)- oder (1S,4R)-1-Amino-4-(hydroxymethyl)-2-cyclopenten der Formeln 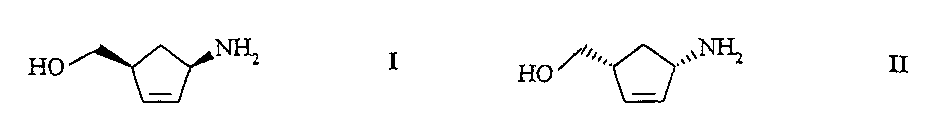 und / oder von (1S,4R)- oder (1R,4S)-Aminoalkoholderivaten der allgemeinen Formeln 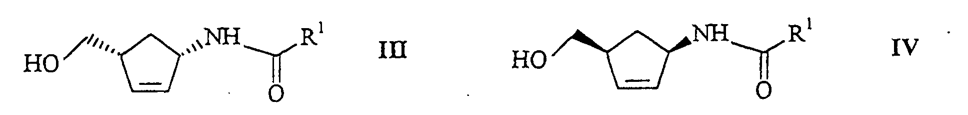 worin R¹ die genannte Bedeutung hat, kann beispielsweise derart durchgeführt werden, daß man in einer ersten Stufe (±)-2-Azabicyclo[2.2.1]hept-5-en-3-on der Formel 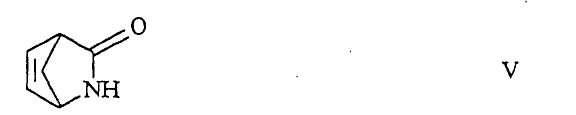 zu einem (±)-2-Azabicyclo[2.2.1]hept-5-en-3-on-derivat der allgemeinen Formel 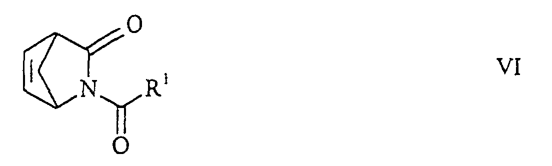 worin R' die genannte Bedeutung hat, acyliert.

Das Edukt (±)-2-Azabicyclo[2.2.1]hept-5-en-3-on kann gemäss der EP-B 0 508 352 hergestellt werden.

Die Acylierung kann mit einem Carbonsäurehalogenid der allgemeinen Formel 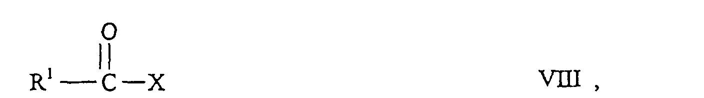 worin X ein Halogenatom bedeutet und R' die genannte Bedeutung hat, oder mit einem Carbonsäureanhydrid der allgemeinen Formel 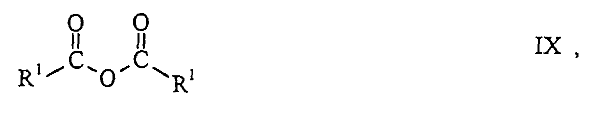 worin R¹ die genannte Bedeutung hat, durchgeführt werden.

Als Halogenatom X können F, Cl, Br oder I verwendet werden. Vorzugsweise wird Cl oder F verwendet.

Beispiele für Carbonsäurehalogenide sind: Acetylchlorid, Chloracetylchlorid, Buttersäurechlorid, Isobuttersäurechlorid, Phenylessigsäurechlorid, Chlorameisensäurebenzylester (Cbz-Cl), Propionsäurechlorid, Benzoylchlorid, Chlorameisensäurealkylester oder tert.-Butyloxycarbonylfluorid. Beispiele für Carbonsäureanhydride sind: tert.-Butoxycarbonylanhydrid, Buttersäureanhydrid, Essigsäureanhydrid oder Propionsäureanhydrid.

Die Acylierung kann ohne Lösungsmittel oder mit einem aprotischen Lösungsmittel durchgeführt werden.
Zweckmässig wird die Acylierung in einem aprotischen Lösungsmittel durchgeführt. Als aprotisches Lösungsmittel sind beispielsweise Pyridin, Acetonitril, Dimethylformamid, Tetrahydrofuran, Toluol, Methylenchlorid, N-Methylpyrrolidon bzw. Mischungen von diesen geeignet. Vorzugsweise wird als Lösungsmittel Pyridin oder Acetonitril, insbesondere eine Mischung aus Pyridin und Acetonitril, verwendet.
Zweckmässig wird die Acylierung bei einer Temperatur von -80 bis 50 °C, vorzugsweise von 0 bis 25 °C, durchgeführt.
In einer zweiten Stufe des Verfahrens kann das (±)-2-Azabicyclo[2.2.1]hept-5-en-3-on-derivat der Formel VI zu-einem Cyclopentenderivat der allgemeinen Formel 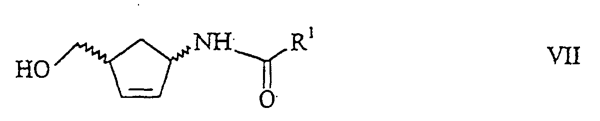 worin R¹ die genannte Bedeutung hat, reduziert werden.

Die Reduktion wird zweckmässig mit einem Alkalimetall- oder Erdalkalimetallborhydrid oder mit einem Alkalimetall- oder Erdalkalimetallaluminiumhydrid oder mit Vitrid (Natrium-bis-(2-methoxyethoxy)aluminiumhydrid) durchgeführt. Als Alkalimetallaluminiumhydrid kann Natrium- oder Kaliumaluminiumhydrid verwendet werden. Als Alkalimetallborhydrid kann Natrium- oder Kaliumborhydrid verwendet werden. Als Erdalkalimetallborhydrid kann Calciumborhydrid verwendet werden.

Zweckmässig wird die Reduktion in einem protischen Lösungsmittel durchgeführt. Als protisches Lösungsmittel können niedere aliphatische Alkohole wie Methanol, Ethanol, Propanol, Isopropanol, Butanol, Isobutanol, sek.-Butanol, tert.-Butanol oder Wasser beziehungsweise Mischungen von diesen verwendet werden.

Die Reduktion wird zweckmässig bei einer Temperatur von -40 bis 40 °C, vorzugsweise von 0 bis 20 °C, durchgeführt.

Die Umsetzung des Cyclopentenderivats der allgemeinen Formel VII zum (1R,4S)- oder (1S,4R)-1-Amino-4-(hydroxymethyl)-2-cyclopenten der Formeln 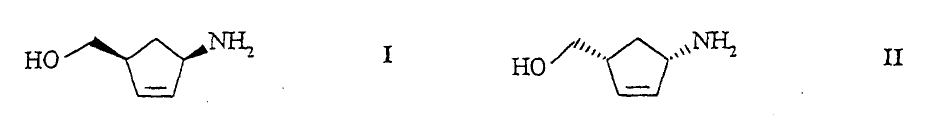 wird erfindungsgemäß entweder mittels Mikroorganismen, mittels Penicillin G Acylasen oder mittels Enzymen mit N-Acetylaminoalkoholhydrolase-Aktivität durchgeführt. Bei dieser Biotransformation fällt neben dem (1R,4S)- oder (1S,4R)-1-Amino-4-(hydroxymethyl)-2-cyclopenten gemäss Formel I oder II, welches gegebenenfalls isoliert wird, das (1S,4R)- oder (1R,4S)-Aminoalkoholderivat der allgemeinen Formeln 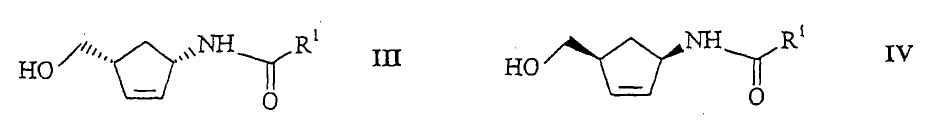 worin R¹ die genannte Bedeutung hat, an. Letztere können gegebenenfalls ebenfalls isoliert werden.

Prinzipiell sind alle Mikroorganismen geeignet, die ein Cyclopentenderivat der allgemeinen Formel VII als einzige Stickstoffquelle, als einzige Kohlenstoffquelle oder als einzige Kohlenstoff- und Stickstoffquelle verwerten. Zweckmässig wird die Biotransformation mit Mikroorganismen durchgeführt, die das (1R,4S)-Isomere des Cyclopentenderivates als einzige Kohlenstoffquelle, als einzige Kohlenstoff- und Stickstoffquelle oder als einzige Stickstoffquelle verwerten.

Vorzugsweise wird die Biotransformation mittels Mikroorganismen der Gattung Alcaligenes / Bordetella, Rhodococcus, Arthrobacter, Alcaligenes, Agrobacterium / Rhizobium, Bacillus, Pseudomonas oder Gordona, insbesondere der Spezies Alcaligenes / Bordetella FB 188 (DSM 11172), Rhodococcus erythropolis CB 101 (DSM 10686), Arthrobacter sp. HSZ 5 (DSM 10328), Rhodococcus sp FB 387 (DSM 11291), Alcaligenes xylosoxydans ssp, denitrificans HSZ 17 (DSM 10329) oder Gordona sp. (DSM 10687), sowie mit deren funktionell aequivalenten Varianten und Mutanten durchgeführt. Diese Mikroorganismen sind wie bereits beschrieben gemäss Budapester Vertrag hinterlegt.

Für das Verfahren ganz besonders geeignet sind die Mikroorganismen-Spezies Alcaligenes / Bordetella FB 188 (DSM 1172), Rhodococcus erythropolis CB 101 (DSM 10686) und Gordona sp. CB 100 (DSM 10687).

Die Biotransformation kann nach üblichem Anzüchten dieser Mikroorganismen mit ruhenden Zellen (nicht wachsenden Zellen, die keine Kohlenstoff- und Energiequelle mehr benötigen) oder mit wachsenden Zellen durchgeführt werden. Vorzugsweise wird die Biotransformation mit ruhenden Zellen durchgeführt.

Die für das Verfahren geeigneten erfindungsgemässen Enzyme, die N-Acetylaminoalkoholhydrolasen, können nach den zuvor beschriebenen Methoden erhalten werden und besitzen die bereits zuvor beschriebenen Eigenschaften.

Geeignete Penicillin G Acylasen werden aus vielen Mikroorganismen wie z. B. Bakterien oder Actinomyceten, im Speziellen aus den folgenden Mikroorganismen, gewonnen: Escherichia coli ATCC 9637, Bacillus megaterium, Streptomyces lavendulae ATCC 13664, Nocardia sp. ATCC 13635, Providencia rettgeri ATCC 9918, Arthrobacter viscosus ATCC 15294, Rhodococcus fascians ATCC 12975, Streptomyces phaeochromogenes ATCC 21289, Achromobacter ATCC 23584 und Micrococcus roseus ATCC 416. Insbesondere werden käufliche Penicillin G Acylasen verwendet wie Penicillin G Acylase EC 3.5.1.11 aus E. coli (Boehringer Mannheim) oder aus Bacillus megaterium.

In einer bevorzugten Ausführungsform werden immobilisierte Penicillin G Acylasen verwendet.

Die Biotransformation kann in fachmännisch üblichen Medien durchgeführt werden, wie beispielsweise in niedermolarem Phosphat-, Citrat- oder Hepes-Puffer, in Wasser, in Vollmedien wie z.B. "Nutrient Yeast Broth" (NYB) oder in dem in Tabelle beschriebenen. Vorzugsweise wird die Biotransformation in dem Medium gemäss Tabelle 1 oder in niedermolarem Phosphatpuffer durchgeführt.

Zweckmässig wird die Biotransformation unter einmaliger oder kontinuierlicher Zugabe des Cyclopentenderivates (Formel VII) so durchgeführt, dass die Konzentration 10 Gew.%, vorzugsweise 2 Gew.% nicht übersteigt.

Der pH kann während der Biotransformation in einem Bereich von 5 bis 9, vorzugsweise von 6 bis 8 liegen. Zweckmässig wird die Biotransformation bei einer Temperatur von 20 bis 40 °C, vorzugsweise von 25 bis 30 °C, durchgeführt.

Wird bei der Biotransformation das (1S,4R)-1-Amino-4-(hydroxymethyl)-2-cyclopenten gebildet, kann dies durch saure Hydrolyse, z. B. mit Salzsäure, in das (1R,4S)-1-Amino-4-(hydroxymethyl)-2-cyclopenten überführt werden.

### Beispiele:

### Beispiel 1

### Herstellung von (±)-2-Acetyl-2-azabicyclo[2.2.1]hept-5-en-3-on

100 g (±)-2-Azabicyclo[2.2.1]hept-5-en-3-on wurden unter Stickstoff in Acetonitril (800 ml) und Pyridin (161,26 ml) gelöst. Bei 12 °C wurden 104,5 g Acetylchlorid über 2 Stunden zugetropft. Das Gemisch wurde noch während 4,5 Stunden bei Raumtemperatur gerührt. Die Mischung wurde mit 800 ml Wasser versetzt und das Acetonitril wurde unter Vakuum eingedampft. Die Wasserphase wurde 3mal mit 400 ml Ethylacetat extrahiert. Die vereinigten org. Phasen wurden noch mit 1N HCl (400 ml), Wasser (400 ml), gesättigtem NaCl (400 ml) gewaschen, mit Magnesiumsulfat getrocknet und voll eingedampft. Der Rückstand wurde in Methylenchlorid aufgenommen und über Kieselgel filtriert. Das Filtrat wurde eingeengt und das Produkt wurde durch Destillation gereinigt. Man erhielt 107,76 g Produkt als klare Flüssigkeit. Die Ausbeute betrug 71 %.
Kp_{0.07 torr} : 51°C
- ¹H-NMR (CDCl₃): δ [ppm]: 2,25 (AB syst., 2H);
- 400 MHz: 2,8 (s, 3H);
3,42 (m, 1H);
5,30 (m, 1H);
6,89 (m, 1H);
6,92 (m, 1 H);

### Beispiel 2

### Herstellung von (±)-2-Butyryl-2-azabicyclo[2.2.1]hept-5-en-3-on

100,3 g (±)-2-Azabicyclo[2.2.1]hept-5-en-3-on wurden unter Stickstoff in Acetonitril (720 ml) und Pyridin (142 ml) gelöst. Bei 12 °C wurden 141,8 g Buttersäurechlorid über 1 Stunde zugetropft. Die Reaktion wurde noch während 3 Stunden bei Raumtemperatur gerührt. Das Gemisch wurde mit 720 ml Wasser versetzt. Das Acetonitril wurde unter Vakuum eingedampft und die Wasserphase wurde 3mal mit Ethylacetat (300 ml) extrahiert. Die vereinigten org. Phasen wurden noch mit 1N HCl (350 ml), gesättigtem NaCl (400 ml) und Wasser (500 ml) gewaschen, mit Magnesiumsulfat getrocknet und voll eingedampft. Das Produkt wurde durch Destillation gereinigt. Man erhielt 107,76 g Produkt als klare Flüssigkeit. Die Ausbeute betrug 85%.
Kp_{0.05 torr} : 70 °C
- ¹H-NMR (CDCl₃): δ [ppm]: 0,98 (t, J = 8.5 Hz, 3H);
- 400 MHz: 1,58 - 1,65 ( 2H);
2,23 (AB syst., 2H);
2,82 - 2,90 ( 2H);
3,42 (m, 1H);
5,30 (m, 1H);
6,62 (m, 1 H);
6,90 (m, 1H).

### Beispiel 3

### Herstellung von (±)-2-Phenylacetyl-2-azabicyclo[2.2.1.]hept-5-en-3-on

33,4 g (±)-2-Azablcyclo[2.2.1]hept-5-en-3-on wurden unter Stickstoff in Acetonitril (240 ml) und Pyridin (48,3 ml) gelöst. Bei 12 °C wurden 68,6 g Phenylessigsäurechlorid über 30 Minuten zugetropft. Die Mischung wurde noch während 3,5 Stunden bei Raumtemperatur gerührt. Das Gemisch wurde mit 240 ml Wasser versetzt. Das Acetonitril wurde unter Vakuum eingedampft und die Wasserphase wurde 3mal mit Ethylacetat (150 ml) extrahiert. Die vereinigten org. Phasen wurden noch mit 1N HCl (150 ml), gesättigtem NaCl (150 ml) und Wasser (150 ml) gewaschen, mit Magnesiumsulfat getrocknet und voll eingedampft.

Das Rohprodukt wurde über Kieselgel abfiltriert (Hexan:Ethylacetat = 1:1). Man erhielt 68,34 g des rohen Produktes als gelbes Öl.

### Beispiel 4

### Herstellung von (±)-2-Propionyl-2-azabicyclo[2.2.1]hept-5-en-3-on

47 g (±)-2-Azabicyclo[2.2.1]hept-5-en-3-on wurden unter Stickstoff in Acetonitril (325 ml) und Pyridin (41 ml) gelöst. Bei 12 °C wurden 43,9 g Propionsäurechlorid über 1 h zugetropft. Die Reaktion wurde noch während 5 h bei Raumtemperatur gerührt. Das Gemisch wurde mit 145 ml Wasser versetzt und das Acetonitril unter Vakuum eingedampft. Die wässrige Phase wurde 3mal mit 115 ml Ethylacetat extrahiert. Die vereinigten organischen Phasen wurden noch mit 1N HCl (140 ml), gesättigtem NaHCO₃ (40 ml) und NaCl (40 ml) Lösungen gewaschen, mit Natriumsulfat getrocknet und vollständig eingedampft. Der Rückstand wurde durch Destillation gereinigt. Man erhielt 55,8 g Titelverbindung, die bei Stehenlassen erstarrt. die Ausbeute betrug 81,6%.
Kp 2,8 mbar 75 - 80 °C
Smp.: 54 -56 °C
- ¹H-NMR (DMSO-d₆): δ [ppm]: 0,95 (t, 3H);
- 400 MHz: 2,10 (quad., 1H);
2,28 (quad., 1H);
2,64 (m, 2H);
3,42 (s, 1H);
5,16 (s, 1H);
6,78 (m, 1H):
6,96 (m, 1H).

### Herstellung von (±)-2-Isobutyryl-2-azabicyclo[2.2.1]hept-5-en-3-on

45,1 g (±)-2-Azabicyclo[2.2.1]hept-5-en-3-on wurden unter Stickstoff in Acetonitril (310 ml) und Pyridin (39 ml) gelöst. Bei 10 °C wurden 54,1 g Isobuttersäurechlorid über 1 h zugetropft. Die Reaktion wurde noch während 5 h bei Raumtemperatur gerührt. Das Gemisch wurde mit 140 ml Wasser versetzt und das Acetonitril unter Vakuum eingedampft. Die wässrige Phase wurde mit 4mal 120 ml Ethylacetat extrahiert. Die vereinigten organischen Phasen wurden noch mit 1N HCl (50 ml) gesättigten NaHCO₃ (50 ml) und NaCl (50 ml) Lösungen gewaschen, mit Natriumsulfat getrocknet und vollständig eingedampft. Der Rückstand wurde in n-Hexan (240 ml) mit Aktivkohle unter Rückfluss gekocht. Nach Filtration der Aktivkohle wurde das Filtrat bei 0 °C gekühlt und die Titelverbindung filtriert. Man erhielt 54,5 g Produkt. Die Ausbeute betrug 76%.
Smp.: 41 - 42 °C
- ¹H-NMR (DMSO-d₆) : δ [ppm]: 0,92 (d, 3H);
- 400 MHz: 1,06 (d, 3H);
2,10(m, 1H);
2,28 (m, 1H);
3,40 (m, 2H);
5,16 (s, 1H);
6,78 (m, 1H);
7,92 (m, 1H).

### Beispiel 6

### Herstellung von (±)-2-Chloracetyl-2-azabicyclo[2.2.1]hept-5-en-3-on

10,1 g (±)-2-Azabicyclo[2.2.1]hept-5-en-3-on wurden unter Stickstoff und bei 10 °C in einer Mischung von Dichlormethan (10 ml), Pyridin (8,4 ml) und 0,22 g N,N-4-dimethylaminopyridin gelöst. 13,5 g Chloracetylchlorid wurden über 1 h zugetropft. Die Temperatur stieg bis 44 °C. Das Gemisch wurde bei Raumtemperatur während 2 h weitergerührt. Die Lösung wurde mit 100 ml Wasser addiert. Nach Phasentrennung wurde die wässrige Phase mit 100 ml Dichlormethan extrahiert. Die vereinigten organischen Phasen wurden mit Natriumsulfat getrocknet und vollständig eingedampft. Der Rückstand wurde in 100 ml Diisopropylether unter Rückfluss in Gegenwart von 1 g Aktivkohle über 10 Minuten gekocht. Nach heisser Filtration wurde das Filtrat bis zur Raumtemperatur abgekühlt, der Feststoff filtriert und getrocknet. Man erhielt 10,35 g Titelverbindung. Die Ausbeute betrug 60%.
Smp.: 86 - 88 °C
- ¹H-NMR (CDCl₃) : δ [ppm]: 2,28 (d, 1H);
- 400 MHz: 2,40 (d, 1H);
3,48 (s, 1H);
4,56 (d, 2H);
5,30 (s, 1H);
6,70 (d, 1H);
6,94 (m, 1H).

### Beispiel 7

### Herstellung von (±)-N-acetyl-1-amino-4-hydroxymethyl-2-cyclopenten

79,56 g (±)-2-Acetyl-2-azabicyclo[2.2.1]hept-5-en-3-on wurden unter Stickstoff in Ethanol (450 ml) gelöst und auf -10 °C gekühlt. 19,8 g Natriumborhydrid wurden portionsweise über 45 Minuten zugegeben.
Die Reaktion wurde bei 0 °C noch 3 Stunden gerührt und dann wurde der pH auf 1,8 mit konz. Schwefelsäure eingestellt. Dieses Gemisch wurde mit Ethylacetat (200 ml) versetzt und die Feststoffe wurden abfiltriert. Dann wurde es voll eingedampft. Der Rückstand wurde in Wasser aufgenommen, mit Methylenchlorid gewaschen und voll eingedampft. Das Rohprodukt wurde noch mit einer Kieselgel Filtration mit Ethylacetat / Methanol 5 : 1 als Lösungsmittel gereinigt. Das Filtrat wurde eingeengt. Man erhielt 51,83 g Produkt als weissen Feststoff. Die Ausbeute betrug 64% bez. eingesetztem 2-Acetyl-2-azabicyclo[2.2.1]hept-5-en-3-on.
Smp. 91 - 93 °C
- ¹H-NMR (DMSO-d₆): δ [ppm]: 1,18 (m, 1H);
- 400 MHz: 1,78 (s, 3H);
2,29 (m, 1 H);
2,66 (m, 1H);
3,35 (s, 2H);
4,58 (s, 1H);
4,72 (m, 1 H);
5,61 (d, 1H);
5,85 (d, 1H);
7,83 (d, 1H).

### Beispiel 8

### Herstellung von (±)-N-butyryl-1-amino-4-hydroxymethyl-2-cyclopenten

73,87 g (±)-2-Butyryl-2-azabicyclo[2.2.1]hept-5-en-3-on wurden unter Stickstoff in Ethanol (400 ml) gelöst und auf -10 °C gekühlt. 15,68 g Natriumborhydrid wurden portionsweise über 45 Minuten zugegeben. Die Reaktion wurde bei 0 °C noch 3 Stunden gerührt und dann wurde der pH auf 1,5 mit konz. Schwefelsäure eingestellt. Dieses Gemisch wurde mit Ethylacetat (200 ml) versetzt und die Feststoffe wurden abfiltriert. Dann wurde es voll eingedampft. Der Rückstand wurde in Wasser aufgenommen, mit Methylenchlorid gewaschen, eingedampft und am Hochvakuum getrocknet. Man erhielt 60,55 g Produkt. Die Ausbeute betrug 80% bez. eingesetztem (±)-2-Butyryl-2-azabicyclo[2.2.1]hept-5-en-3-on.
Smp. 71 - 72 °C
- ¹H-NMR (CDCl₃) : δ [ppm]: 0,98 (t, J = 8,5 Hz, 3H);
- 400 MHz: 1,40 - 1,50 (1H);
1,58 - 1,68 (2H);
2,10 - 2,18 (2H);
2,42 - 2,55 ( 1H);
2,85 (m, 1H);
3,62 (AB syst., 2H);
4,98 (m, 1H);
5,78-5.82 (2H);
6,38 (m, 1H).

### Beispiel 9

### Herstellung von (±)-N-phenylacetyl-1-amino-4-hydroxymethyl-2-cyclopenten

67 g rohes (±)-2-Phenylacetyl-2-azabicyclo[2.2.1]hept-5-en-3-on wurden unter Stickstoff in Ethanol (450 ml) gelöst und auf -10 °C gekühlt. 13,2 g Natriumborhydrid wurden portionsweise über 1 Stunde zugegeben. Die Reaktion wurde bei Raumtemperatur noch 3,5 Stunden gerührt und dann wurde der pH auf 3,8 mit konz. Schwefelsäure eingestellt. Das Gemisch wurde voll eingedampft. Der Rückstand wurde getrocknet und mit einer Kieselgel Filtration (Hexan:Ethylacetat = 1 : 9) gereinigt. Man erhielt nach Umkristallisation aus Ethylacetat 54,6 g als weissen Feststoff. Die Ausbeute betrug 80% bez. eingesetztem (±)-2-Phenylacetyl-2-azabicyclo[2.2.1]hept-5-en-3-on.
- ¹H-NMR (CDCl₃) : δ [ppm]: 1,28 - 1,35 (1H);
- 400 MHz: 1,40 (m, 1 H);
2,38 - 2,45 (1H);
2,79 (m, 1H);
3,50 (AB syst., 2H);
3,52 (s, 3H);
4,98 (m, 1H);
5,75 (m, 2H);
5,98 (m, 1H);
7,20 - 7,38 (5H).

### Beispiel 10

### Herstellung von (±)-N-BOC-1-amino-4-hydroxymethyl-2-cyclopenten

15 g rohes (±)-1-Amino-4-hydroxymethyl-2-cyclopenten-hydrochlorid wurden unter Stickstoff bei Raumtemperatur in einer Mischung von 150 ml Wasser und 150 ml Dioxan gelöst. Die Lösung wurde mit 1N NaOH auf pH 14 gestellt, dann mit einer Diethylether-Lösung von tert-Butyloxycarbonyl-fluorid (BOC-F, 20% Überschuss) versetzt und bei Zimmertemperatur noch 3 h weitergerührt (BOC-F laut Synthesis 1975, 599 hergestellt). Der pH wurde mit konz. HCl auf 2 gestellt. Nach Destillation von den organischen Lösungsmitteln, wurde zum Rückstand 50 ml Wasser zugegeben und das Gemisch mit 3mal 100 ml Ethylacetat extrahiert. Die vereinigten organischen Phasen wurden vollständig eingedampft. Der Rückstand wurde in einer Mischung von 110 ml Diisopropylether und 80 ml n-Hexan kristallisiert. Man erhielt 11,95 g Titelverbindung. Die Ausbeute betrug 56%.
Smp.: 68 -70 °C
- ¹H-NMR (DMSO-d₆): δ [ppm]: 1,18 (m, 1H);
- 400 Mhz: 1,38 (s, 9H);
2,26 (m, 1H);
2,65 (m, 1H);
3,33 (t, 2H);
4,45 (m, 1H);
4,55 (t, 1H);
5,62 (m, 1H);
5,79 (m, 1H);
6,73 (d, 1H).

### Beispiel 11

### Herstellung von (±)-N-propionyl-1-amino-4-hydroxymethyl-2-cyclopenten

16,6 g (±)-2-Propionyl-2-azabicyclo[2.2.1]hept-5-en-3-on wurden unter Stickstoff in Wasser (140 ml) und 2-Butanol (66 ml) gelöst und auf -5 °C gekühlt. 3 g Natriumborhydrid wurden portionsweise über 2 h zugegeben. Das Gemisch wurde bei 10 °C noch 2,5 h gerührt und dann wurde mit einer Mischung von konz. Salzsäure und Wasser (1/1) auf pH 2,2 eingestellt. Die Lösung wurde auf 40 g eingedampft und auf pH 6,2 mit 2N NaOH eingestellt. Das Gemisch wurde mit 5mal 50 ml Dichlormethan extrahiert. Die vereinigten organischen Phasen wurden vollständig eingedampft und der Rückstand in Toluol (150 ml) umkristallisiert. Man erhielt 11,1 g Titelverbindung. Die Ausbeute betrug 65%.
Smp.: 67 - 68 °C
- ¹H-NMR (DMSO-d₆): δ [ppm]: 0,96 (t, 3H);
- 400 MHz: 1,16 (quint., 1H);
2,04 (quad., 2H);
2,26 (m, 1H);
2,66 (m, 1H);
3,34 (m, 2H);
4,58 (t, 1H);
4,72 (m, 1H);
5,61 (m, 1H);
5,84 (m, 1H);
7,72 (d, 1H).

### Beispiel 12

### Herstellung von (±)-N-isobutyryl-1-amino-4-hydroxymethyl-2-cyclopenten

9 g (±)-2-Isobutyryl-3-azabicyclo[2.2.1]hept-5-en-3-on wurden unter Stickstoff in Wasser (32 ml) und 2-Butanol (84 ml) gelöst und auf 0 °C gekühlt. 1,37 g Natriumborhydrid wurden portionsweise über 3,5 h zugegeben. Das Gemisch wurde bei 20 °C 3 h weitergerührt, dann wurde es mit einer Mischung von konz. Salzsäure und Wasser (1/1) auf pH 2,5 eingestellt und dann mit 2 N NaOH neutralisiert. Die Lösung wurde auf 40 g eingedampft. Der Rückstand wurde mit 3mal 80 ml Dichlormethan extrahiert. Die vereinigten organischen Phasen wurden vollständig eingedampft. Der erhaltene Feststoff wurde in 25 ml Toluol kristallisiert. Man erhielt 6,8 g Titelverbindung. Die Ausbeute betrug 73,6%.
Smp.: 80 - 81 °C
- ¹H-NMR (DMSO-d₆) : δ [ppm]: 0,98 (d, 6H);
- 400 MHz: 1,16 (quint., 1H);
2,30 (m, 2H);
2,68 (m, 1H);
3,32 (t, 2H);
4,58 (t, 1H);
4,70 (m, 1H);
5,61 (m, 1H);
5,82 (m, 1 H);
7,68 (d, 1H).

### Beispiel 13

### Herstellung von (1R,4S)-1-amino-4-(hydroxymethyl)-2-cyclopenten mittels Penicillin G Acylasen

Für die Biotransformation wurde Penicillin G Acylase EC 3.5.1.11 aus E.coli (Boehringer Mannheim) 165 U (Units)/g oder Penicillin G Acylase EC 3.5.1.11 aus Bacillus megaterium eingesetzt.
Hierzu wurde 50 mM Natriumphosphatpuffer (pH 5 - 9; 4 ml) mit 1 Gew. % nicht racemisches N-Phenacetyl-1-amino-4-hydroxymethyl-2-cyclopenten und 400 mg der entsprechenden Penicillin G Acylase bei 37 °C inkubiert.
Nach bestimmten Zeitintervallen wurden Proben entnommen und mittels Dünnschichtchromatographie (Kieselgel 60, Butanol:Wasser:Eisessig =3:1:1; Detektion mit Ninhydrin), Gaschromatographie (Kapillarsäule, HP-5, 5% Phenylmethylsiloxan) oder HPLC analysiert. Das Enzym spaltete mit hoher Aktivität die Phenacetylgruppe ab und setzte dabei bis zu 40% den entsprechenden Aminoalkohol frei. Der freie Aminoalkohol wurde mit einem ee-Wert von 80% erhalten.

### Beispiel 14

### Herstellung von (1R,4S)-1-Amino-4-(hydroxymethyl)-2-cyclopenten mittels Mikroorganismen

14.1 Klärschlamm (20 %) aus der Kläranlage ARA in Visp wurde in dem A + N-Medium (siehe Tabelle 1), enthaltend 0,5 Gew.% N-Acetyl-, N-Propionyl, N-Isobutyryl-, oder N-Butyryl-1-amino-4-hydroxymethyl-2-cyclopenten bei 37 °C unter Schütteln inkubiert. Die Bildung von (1R,4S)-1-Amino-4-(hydroxymethyl)-2-cyclopenten wurde mittels Dünnschichtchromatographie verfolgt.
1% dieser Anreicherungen wurden 1-3 mal überimpft und auf Festmedien (Plate Count Agar im Medium von Tabelle 1; 20 g/l) vereinzelt. Auf diese Weise wurden die Mikroorganismen Alcaligenes / Bordetella FB 188 (DSM 1172), Rhodococcus erythropolis CB 101 (DSM. 10686), Gordona sp. CB 100 (DSM 10687) und Rhodococcus sp. FB 387 (DSM 11291) isoliert.
14.2 Die auf diese Weise isolierten Mikroorganismen wurden im Medium (Tabelle 1), enthaltend 0,5% N-Acetyl-, N-Propionyl-, N-Isobutyryl oder N-Butyryl-1-amino-4-hydroxymethyl-2-cyclopenten gezüchtet. Sie wuchsen 24 bis 36 Stunden bis zu einer optischen Dichte (OD) von 2 bis 3. Die dabei erhaltenen Zellen wurden in der spätexponentiellen Wachstumsphase geerntet und in 10 mM Phosphatpuffer gewaschen.
Die anschliessende Biotransformation wurde in 50 mM Phosphatpuffer (pH 4,5 -9), enthaltend 1 Gew.% N-Acetyl-, N-Isobutyryl oder N-Butyryl-1-amino-4-hydroxymethyl-2-cyclopenten durchgeführt. Nach Dünnschichtchromatographie wurde festgestellt, dass 50 % des Substrates zu (1R,4S)-1-Amino-4-(hydroxymethyl)-2-cyclopenten hydrolysiert waren. HPLC-Analysen ergaben ee-Werte zwischen 80 und 93%.
Wurde als Substrat N-Butyryl-1-amino-4-hydroxymethyl-2-cyclopenten eingesetzt, betrug die Biotransformationsrate 0,14 (g / 1 / h /OD) für den Stamm DSM 10686, wenn die Anzucht auf A + N-Medium erfolgte und 0,03 (g / 1 / h / OD), wenn die Anzucht auf NYB ("Nutrient Yeast Broth")-Medium enthaltend N-Butyryl-1-amino-4-hydroxymethyl-2-cyclopenten erfolgte.
Wurde die gleiche Umsetzung mit dem Stamm DSM 10687 bei einer Substratkonzentration (N-Butyryl-1-amino-4-hydroxymethyl-2-cyclopenten) von 200 mM durchgeführt, betrug die Biotransformationsrate 0,161 (g / 1 / h / OD).

**Tabelle 1**

| **A + N-Medium** | |
|---|---|
| MgCl₂ | 0,4 g/l |
| CaCl₂ | 0,014 g/l |
| FeCl₃ | 0,8 mg/l |
| Na₂SO₄ | 0,1 g/l |
| KH₂PO₄ | 1 g/l |
| Na₂HFO₄ | 2,5 g/l |
| NaCl | 3 g/l |
| Vitaminlösung | 1 ml/l |
| Spurenelementlösung | 1 ml/l |
| pH 7,5 | |

14.3 Rhodococcus erythropolis DSM 10686 wurde in einem 6 1 Fermenter in Minimalmedium (vgl. Tabelle 2) mit Ammoniumacetat (3 g/l) als Kohlenstoff- bzw. Stickstoff-Quelle bei 30 °C auf eine Zelldichte von OD 650 > 25 angezogen. Während des Zellwachstums wurde kontinuierlich 50% Essigsäure als zusätzliche C-Quelle zugegeben. Um die enzymatische Aktivität zu induzieren, wurden dann 60 g (+/-) N-Acetyl-1-amino-4-hydroxymethyl-2-cyclopenten zugesetzt und für eineige Stunden weiter inkubiert. Schliesslich wurden noch einmal 40 g (+/-)-N-Acetyl-1-amino-4-hydroxymethyl-2-cyclopenten zugegeben und dann weitere zehn Stunden inkubiert. Der Verlauf der Biotransformation wurde on-line mit HPLC verfolgt. Mit dem Erreichen von 40% analytischer Ausbeute, bezogen auf das eingesetzte racemische Substrat, und einem ee-Wert von 85% wurde die Fermentation durch Säurezugabe abgebrochen.

**Tabelle 2**

| Medienzusammensetzung | |
|---|---|
| **Komponente** | **Konzentration** |
| Hefeextrakt | 0,5 g/l |
| Pepton M66 | 0,5 g/l |
| KH₂PO₄ | 4,0 g/l |
| Na₂HPO₄.2H₂O | 0,5 g/l |
| K₂SO₄ | 2,0 g/l |
| NH₄-acetat | 3,0 g/l |
| CaCl₂ | 0,2 g/l |
| MgCl₂·6H₂O | 1,0 g/l |
| Spurenelemente-Lösung (siehe unten) | 1,5 ml/l |
| PPG (Polypropylenglycol) | 0,1 g/l |
| | |

| **Spurenelemente-Lösung** | |
|---|---|
| KOH | 15,1 g/l |
| EDTA·Na₂·2H₂O | 100,0 g/l |
| ZnSO₄·7H₂O | 9,0 g/l |
| MnCl₂·4H₂O | 4,0 g/l |
| H₃BO₃ | 2,7 g/l |
| CoCl₂·6H₂O | 1,8 g/l |
| CuCl₂·2H₂O | 1,5 g/l |
| NiCl₂·6H₂O | 0,18 g/l |
| Na₂MoO₄·2H₂O | 0,27 g/l |

14.4 Analog zu Beispiel 14.3 wurden die Mikroorganismen Arthrobacter sp. HSZ 5 (DSM 10328), Rhodococcus sp FB387 (DSM 11291) und Alcaligenes xylosoxydans ssp. denitrificans HSZ 17 (DSM 10329) auf Natriumacetat im Medium (Tabelle 1) mit und ohne N-Acetyl-, N-Propionyl, N-Isobutyryl- oder N-Butyryl-1-amino-4-hydroxymethyl-2-cyclopenten, im folgenden als Aminoalkohole abgekürzt, angezogen.
Folgende Ergebnisse wurden mit exponentiellen Zellen, die ohne Aminoalkohole angezogen wurden, erzielt (HPLC-analysiert):

| **Stamm** | **Rate [mMol/OD . h]** | **ee-Wert/Umsatz [%]** |
|---|---|---|
| HSZ 5 (DSM 10328) | 0,05 | 88,7/16 |
| HSZ 17 (DSM 10329) | 0,005 | 95/23 |
| CB101 (DSM 10686) | 0,1 | 84/39 |

Von allen Ansätzen wurden exponentielle und stationäre Zellen geerntet und als ruhende Zellen zur Biotransformation eingesetzt. Es konnte aufgrund der DC-Analyse kein Unterschied von mit Aminoalkohol induzierten oder nicht induzierten Zellen in der Anfangsrate beobachtet werden.

### Beispiel 15

### Reinigung der N-Acetylaminoalkoholhydrolase aus Rhodococcus erythropolis CB101 (DSM 10686)

Das Enzym wurde wie nachstehend beschrieben gereinigt bis nur noch eine Proteinbande im SDS-PAGE (Pharmacia Phast Gel, 10-15 % Gradient) bei einem Molekulargewicht von 50 kD beobachtet wurde.

Zellen von Rhodococcus erythropolis CB101 (DSM 10686) wurden in 50 mM Tris-Puffer (pH 6,2) gewaschen und auf eine optische Dichte von 190 bei OD₆₅₀ₙₘ konzentriert. Nach Zugabe von Phenylmethansulfonylfluorid (PMSF) auf eine Endkonzentration von 1 mM und DNAse wurden die Zellen mit der French-Press behandelt, um ein Rohextrakt zu erhalten. Nach Zentrifugieren wurden 200 ml zellfreies Extrakt mit einer Proteinkonzentration von 4,8 mg ml⁻¹ erhalten.
960 mg des zellfreien Extraktes wurden auf eine HiLoad 26/10 Q-Sepharose-Ionenaustausch-Chromatographie-Säule (Pharmacia) aufgetragen, die mit einem 50 mM Tris-Puffer (pH 8,0), enthaltend 1 mM Dithiothreitol (DTT), equilibriert worden war:

Nach Waschen der Säule mit dem gleichen Puffer wurden die Proteine mit einem linearen Puffer-Gradienten (1500 ml; Gradient: 50 mM Tris-Puffer (pH 8,0), enthaltend 1 mM DTT - 50 mM Tris-Puffer (pH 7,0), enthaltend 1 mM DTT und 1 M NaCl) eluiert. Das Enzym eluierte von der Säule zwischen 370 und 430 mM NaCl und bei einem pH von 7,6. Die aktiven Fraktionen wurden gesammelt und auf 9 ml konzentriert. Der Proteingehalt betrug 41 mg.
Zur weiteren Reinigung wurde die Proteinlösung auf eine Geifiltrationschromatographie-Säule HiLoad 26/60 Superdex 75 (Pharmacia) aufgetragen, die mit einem 50 mM Tris-Puffer, enthaltend 50 mM NaCl und 1 mM DTT, equlibriert worden war. Die aktiven Fraktionen wurden vereinigt und hatten einen Gesamtproteingehalt von 10,9 mg.
Diese Proteinlösung wurde auf eine Mono Q HR5/5 Säule (Pharmacia) aufgetragen, die mit 50 mM Tris-Puffer (pH 8,5), enthaltend 1 mM DTT, equilibriert worden war. Die Proteine wurden mit einem linearen Gradienten (40 ml) von 50 mM Tris-Puffer (pH 8,5), enthaltend 1 mM DTT - 50 mM Tris-Puffer (pH 8,5), enthaltend 1 mM DTT und 1 M NaCl eluiert. Das Enzym eluierte zwischen 390 mM NaCl und 440 mM NaCl. Die aktiven Fraktionen enthielten 1,4 mg Protein.

Im letzten Reinigungsschritt wurde die gleiche Säule, equilibriert mit dem gleichen Puffer, verwendet. Als Elutionsgradient diente dergleiche Puffer mit 0 - 500 mM NaCl und pH 7,0 - 8,5. Auf diese Weise konnten 430 µg reines Enzym isoliert werden.

Die N-terminale Sequenz des Enzyms wurde direkt vom "Protein-Blot" bestimmt. Es wurde eine Sequenz von den 20 folgenden Aminosäuren erhalten: Thr-Glu-Gln-Asn-Leu-His-Trp-Leu-Ser-Ala Thr-Glu-Met-Ala-Ala-Ser-Val-Ala-Ser-Asn.
Diese Sequenz zeigt keine Homologie zu bekannten Proteinen.

### Beispiel 16

### Enzymcharakterisierung

Die Enzymcharakterisierung wurde sowohl mit gereinigtem Enzym als auch mit zellfreiem Extrakt, das mittels einer Sephadex G-25 Säule (PD-10, Pharmacia) entsalzt war, durchgeführt.
Die Proteinkonzentration im zellfreien Extrakt betrug 7,3 mg ml⁻¹ und die Proteinkonzentration des gereinigten Enzyms betrug 135 ug ml⁻¹. PMSF wurde zum zellfreien Extrakt nicht zugegeben.
16.1 Kₘ-Bestimmung
   Die Kₘ-Bestimmung wurde im zellfreien Extrakt durchgeführt. Der Kₘ-Wert der Reaktion betrag bei pH 7,0 und bei einer Temperatur von 30 °C 22,5 mM für Substrat N-acetyl-1-amino-4-hydroxymethylcyclopenten.
16.2 pH-Optimum
   Das pH-Optimum für die Hydrolyse von N-acetyl-1-amino-4-hydroxymethyl-2-cyclopenten (25 mM) wurde mit dem gereinigten Enzym und in zellfreiem Extrakt in einem pH-Bereich von pH 6,2 - 9,0 in folgenden Pufferlösungen bestimmt.
   Tris-Puffer 100 mM pH 9,0; 8,5; 8,0; 7,5; 7,0
   Citrat-Phosphat-Puffer 100 mM pH 7,0; 6,55; 6,2
   Die Aktivität wurde 24 h lang gemessen.
   Das pH-Optimum für die Reaktion war zwischen pH 7,0 und pH 7,5 für die Produktion des 1R,4S und des 1S,4R Enantiomeren.
   Im zellfreien Extrakt lag das pH-Optimum für die Aktivität bei pH 7,0. Die Selektivität war jedoch besser zwischen pH 6,0 und pH 7,0.

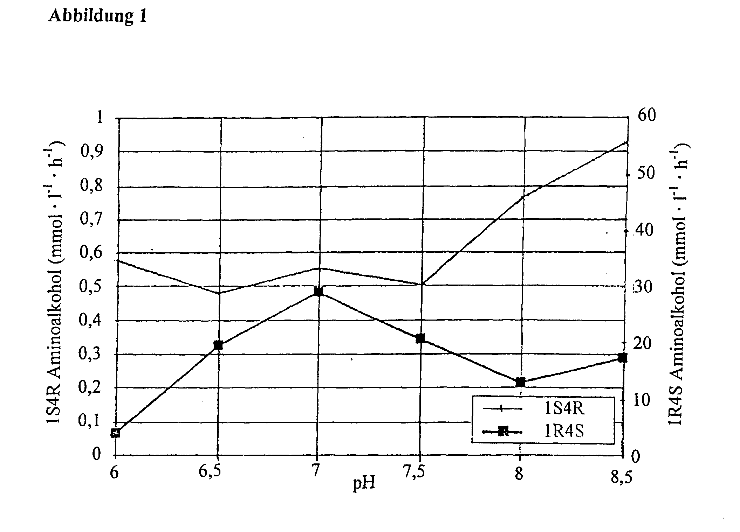

   Abbildung 1 zeigt die Aktivität der N-Acetylaminoalkoholhydrolase (zellfreies Extrakt) aus Rhodococcus erythropolis CB 101 (DSM 10686) in Abhängigkeit vom pH-Wert.
16.3 Das Temperaturoptimum für die in Beispiel 16.2 angegebene Reaktion lag zwischen 25 und 30 °C.

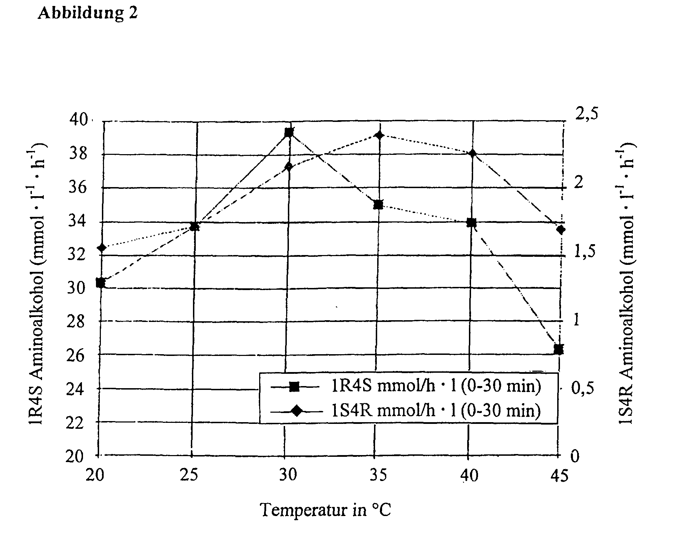

   Abbildung 2 zeigt die Aktivität der N-Acetylaminoalkoholhydrolase (zellfreies Extrakt) aus Rhodococcus erythropolis CB 101 (DSM 10686) in Abhängigkeit von der Temperatur.

## Patentansprüche

1. Mikroorganismen, welche befähigt sind Cyclopentenderivate, ausgewählt aus Verbindungen der allgemeinen Formel 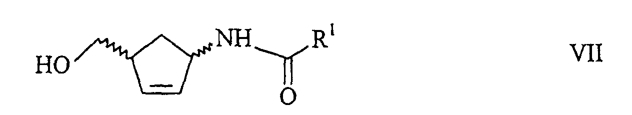 worin R¹ C₁-C₄-Alkyl, C₁-C₄-Alkoxy, Aryl oder Aryloxy bedeutet, als einzige Stickstoffquelle, als einzige Kohlenstoffquelle oder als einzige Kohlenstoff- und Stickstoffquelle zu verwerten, **dadurch gekennzeichnet, dass** die Mikroorganismen ausgewählt sind aus den Spezies Alcaligenes / Bordetella FB 188 (DSM 1172), Rhodococcus erythropolis CB 101 (DSM 10686), Arthrobacter sp. HSZ 5 (DSM 10328), Rhodococcus sp. FB 387 (DSM 11291), Alcaligenes xylosoxydans ssp. denitrificans HSZ 17 (DSM 10329) oder Gordona sp. CB 100 (DSM 10687).

2. Enzym mit N-Acetylaminoalkoholhydrolase-Aktivität, erhältlich aus Mikroorganismen, welche befähigt sind, wenigstens ein Cyclopentenderivat, ausgewählt aus den Verbindungen der allgemeinen Formel 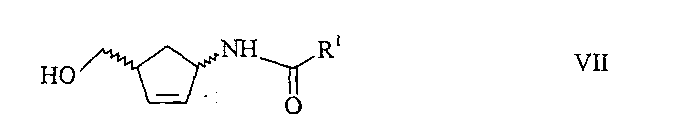 worin R¹ die in Anspruch 1 angegebene Bedeutung hat, als einzige Stickstoffquelle, als einzige Kohlenstoffquelle oder als einzige Kohlenstoff- und Stickstoffquelle zu verwerten, wobei das Enzym befähigt ist, die Verbindung der Formel VII zu hydrolysieren und **gekennzeichnet ist durch**
a) eine N-terminale Aminosäuresequenz von Thr-Glu-Gln-Asn-Leu-His-Trp-Leu-Ser-Ala-Thr-Glu-Met-Ala-Ala-Ser-Val-Ala-Ser-Asn; und
b) ein Molekulargewicht, bestimmt nach 10-15% SDS-PAGE, von 50 kD.

3. Enzym nach Anspruch 2, femer **gekennzeichnet durch**
c) ein pH-Optimum von pH 7,0 ± 1,0,
d) ein Temperatur-Optimum zwischen 25 °C und 30 °C bei einem pH-Wert von 7,0; und
e) einen K_{M}-Wert für das Substrat N-Acetyl-1-amino-4-hydroxymethyl-2-cyclopenten von 22,5 mM ± 7,5 mM (30 °C, 100 mM Phosphat Puffer).

4. Enzym nach Anspruch 2 oder 3, erhältlich aus Mikroorganismen der Gattung Rhodococcus, Gordona, Arthrobacter, Alcaligenes, Agrobacterium / Rhizobium, Bacillus, Pseudomonas oder Alcaligenes / Bordetella.

5. Enzym nach einem der Ansprüche 2 bis 4, erhältlich aus Mikroorganismen, die ausgewählt sind aus den Spezies Alcaligenes / Bordetella FB 188 (DSM 1172), Rhodococcus erythropolis CB 101 (DSM 10686), Arthrobacter sp. HSZ 5 (DSM 10328), Rhodococcus sp. FB 387 (DSM 11291), Alcaligenes xylosoxydans ssp. denitrificans HSZ 17 (DSM 10329) oder Gordona sp. CB 100 (DSM 10687).

6. Verfahren zur Herstellung von (1R,4S)- oder (1S,4R)-1-Amino-4-(hydroxymethyl)-2-cyclopenten der Formeln I und II 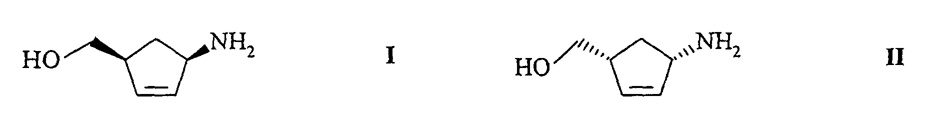 und / oder von (1S,4R)- oder (1R,4S)-Aminoalkoholderivaten der allgemeinen Formeln II und IV 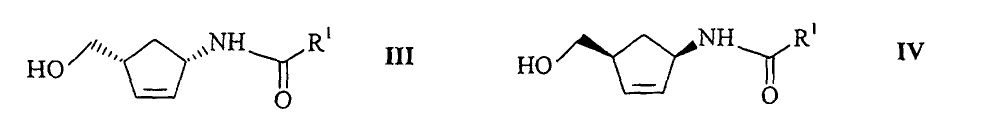 worin R¹ C₁-C₄-Alkyl, C₁-C₄-Alkoxy, Aryl oder Aryloxy bedeutet, umfassend die Umsetzung eines Cyclopentenderivats der allgemeinen Formel 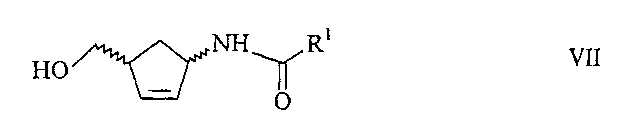 worin R¹ die genannte Bedeutung hat, mittels eines Mikroorganismus, der befähigt ist, die Verbindung der obigen allgemeinen Formel VII als einzige Stickstoffquelle, als einzige Kohlenstoffquelle oder als einzige Kohlenstoff- und Stickstoffquelle zu verwerten, eines Enzyms mit Acetylaminohydrolase-Aktivität, das aus solchen Mikroorganismen erhältlich ist und befähigt ist, die Verbindung der obigen allgemeinen Formel VII zu hydrolysieren, und/oder einer Penicillin G Acylase in das (1R,4S)- oder (1S,4R)-1-Amino-4-(hydroxymethyl)-2-cyclopenten gemäss Formel I oder II, sowie gegebenenfalls Isolierung dieser Verbindungen und/oder der neben dem (1R,4S)- oder dem (1S,4R)-1-Amino-4-(hydroxymethyl)-2-cyclopenten anfallenden (1S,4R)- oder (1R,4S)-Aminoalkoholderivate der Formeln III oder IV.

7. Verfahren nach Anspruch 6, **dadurch gekennzeichnet, dass** das Cyclopentenderivat der allgemeinen Formel 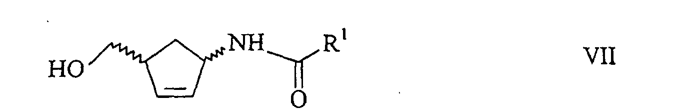 worin R¹ die genannte Bedeutung hat, dadurch hergestellt wird, daß in einer ersten Stufe (±)-2-Azabicyclo[2.2.1]hept-5-en-3-on der Formel 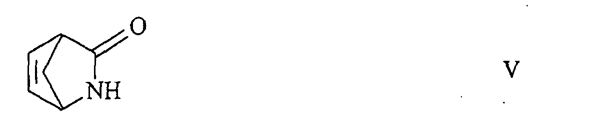 zu einem (±)-2-Azabicyclo[2.2.1]hept-5-en-3-on-derivat der allgemeinen Formel 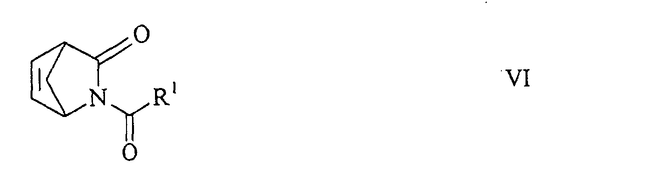 worin R¹ die genannte Bedeutung hat, acyliert wird, und diese Verbindung in einer zweiten Stufe zu dem Cyclopentenderivat der allgemeinen Formel VII reduziert wird.

8. Verfahren nach Anspruch 7, **dadurch gekennzeichnet, dass** man die Acylierung in der ersten Stufe mit einem Carbonsäurehalogenid der allgemeinen Formel 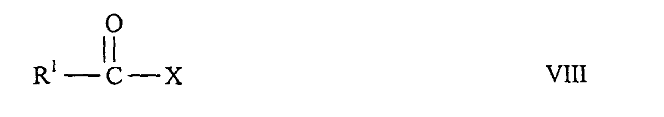 worin X ein Halogenatom bedeutet und R¹ die genannte Bedeutung hat, oder mit einem Carbonsäureanhydrid der allgemeinen Formel 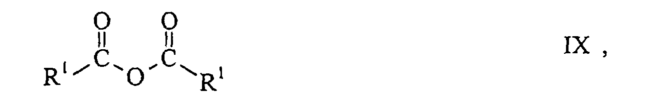 worin R1 die genannte Bedeutung hat, durchführt.

9. Verfahren nach einem der Ansprüche 7 oder 8, **dadurch gekennzeichnet, dass** man die Acylierung in der ersten Stufe in einem aprotischen Lösungsmittel durchführt.

10. Verfahren nach einem der Ansprüche 7 bis 9, **dadurch gekennzeichnet, dass** man die Reduktion in der zweiten Stufe mit einem Alkali- oder Erdalkalimetallborhydrid, einem Alkali- oder Erdalkalimetallaluminiumhydrid und/oder mit Vitrid durchführt.

11. Verfahren nach einem der Ansprüche 7 bis 10, **dadurch gekennzeichnet, dass** man die Reduktion in der zweiten Stufe in einem protischen Lösungsmittel durchführt.

12. Verfahren nach einem der Ansprüche 6 bis 11, **dadurch gekennzeichnet, dass** die Umsetzung des Cylopentenderivats der allgemeinen Formel VII mittels Mikroorganismen der Gattung Rhodococcus, Gordona, Arthrobacter, Alcaligenes, Agrobacterium / Rhizobium, Bacillus, Pseudomonas oder Alcaligenes / Bordetella erfolgt.

13. Verfahren nach einem der Ansprüche 6 bis 12, **dadurch gekennzeichnet, dass** die Umsetzung des Cylopentenderivats der allgemeinen Formel VII mittels einer Penicillin G Acylase aus Mikroorganismen der Spezies Bacillus megaterium oder Escherichia coli erfolgt.

14. Verfahren nach einem der Ansprüche 6 bis 13, **dadurch gekennzeichnet, dass** man die Umsetzung in der dritten Stufe bei einer Temperatur von 20 bis 40 °C und bei einem pH-Wert von 5 bis 9 durchführt.

## Claims

1. Microorganisms, which are rendered capable of using cyclopentene derivatives selected from compounds having the general formula 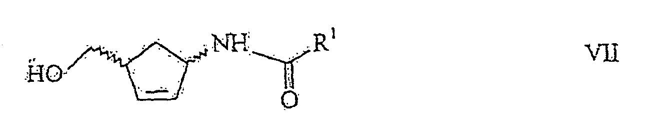 in which R¹ stands for C₁-C₄-alkyl, C₁-C₄-alkoxy, aryl or aryloxy, as the sole source of nitrogen, the sole source of carbon or the sole source of carbon and nitrogen, **characterised in that** the microorganisms are selected from the species Alcaligenes/Bordetella FB 188 (DSM 1172), Rhodococcus erythropolis CB 101 (DSM 10686), Arthrobacter sp. HSZ 5 (DSM 10328), Rhodococcus sp. FB 387 (DSM 11291), Alcaligenes xylosoxydans ssp. denitrificans HSZ 17 (DSM 10329) or Gordona sp. CB 100 (DSM 10687).

2. Enzyme with N-acetylamino alcohol hydrolase activity, which can be obtained from microorganisms rendered capable of using at least one cyclopentene derivative selected from compounds having the general formula 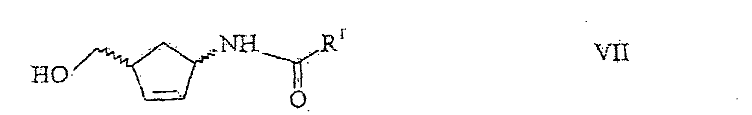 in which R¹ has the meaning specified in claim 1, as the sole source of nitrogen, the sole source of carbon or the sole source of carbon and nitrogen, whereby the enzyme is rendered capable of hydrolysing the compound of formula VII and **characterised by**
a) an N-terminal amino acid sequence of Thr-Glu-Gln-Asn-Leu-His-Trp-Leu-Ser-Ala-Thr-Glu-Met-Ala-Ala-Ser-Val-Ala-Ser-Asn; and
b) a molecular weight of 50 kD determined on the basis of 10-15% SDS-PAGE.

3. Enzymes as claimed in claim 2, further **characterised by**
c) a pH optimum of pH 7.0 ± 1.0,
d) a temperature optimum of between 25°C and 30°C at a pH value of 7.0; and
e) a K_{M} value for the N-acetyl-1-amino-4-hydroxymethyl-2-cyclopentene substrate of 22.5 mM ± 7.5 mM (30°C, 100 mM phosphate buffer).

4. Enzyme as claimed in claim 2 or 3, which can be obtained from microorganisms of the genus Rhodococcus, Gordona, Arthrobacter, Alcaligenes, Agrobacterium/Rhizobium, Bacillus, Pseudomonas or Alcaligenes/Bordetella.

5. Enzyme as claimed in one of claims 2 to 4, which can be obtained from microorganisms selected from the species Alcaligenes/Bordetella FB 188 (DSM 1172), Rhodococcus erythropolis CB 101 (DSM 10686), Arthrobacter sp. HSZ 5 (DSM 10328), Rhodococcus sp. FB 387 (DSM 11291), Alcaligenes xylosoxydans ssp. denitrificans HSZ 17 (DSM 10329) or Gordona sp. CB 100 (DSM 10687).

6. Method of preparing (1R,4S)- or (1S,4R)-1-amino-4-(hydroxymethyl)-2-cyclopentene of formulas I and II 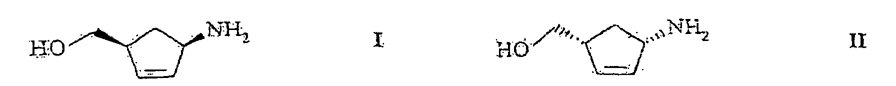 and/or (1S,4R)- or (1R,4S)-amino alcohol derivatives having the general formulas III and IV 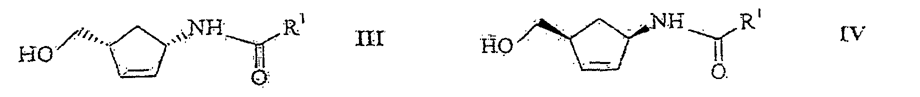 in which R¹ stands for C₁-C₄-alkyl, C₁-C₄-alkoxy, aryl or aryloxy, which consists of reacting a cyclopentene derivative having the general formula 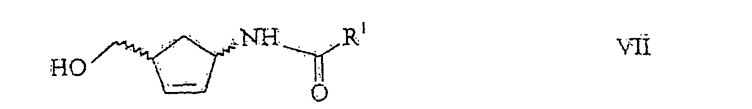 in which R¹ has the specified meaning, by means of a microorganism which is rendered capable of using the compound having general formula VII above as the sole source of nitrogen, the sole source of carbon or the sole source of carbon and nitrogen, an enzyme with acetylamino hydrolase activity which can be obtained from such microorganisms and rendered capable of hydrolysing the compound having the general formula VII above, and/or a Penicillin G acylase in (1R,4S)- or (1S,4R)-1-amino-4-(hydroxymethyl)-2-cyclopentene having formula I or II, and optionally isolating these compounds and/or the (1S,4R)- or (1R,4S)-amino alcohol derivatives of formulas III or IV which occur in addition to (1R,4S)- or (1S,4R)-1-amino-4-(hydroxymethyl)-2-cyclopentene.

7. Method as claimed in claim 6, **characterised in that** the cyclopentene derivative having the general formula 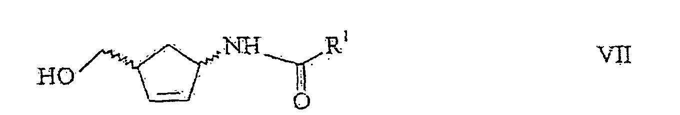 in which R¹ has the specified meaning, is prepared, whereby in a first stage (±)-2-azabicyclo[2.2.1]hept-5-en-3-one having formula 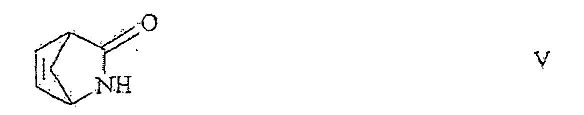 is acylated to obtain a (±)-2-azabicyclo[2.2.1]hept-5-en-3-one derivative having the general formula 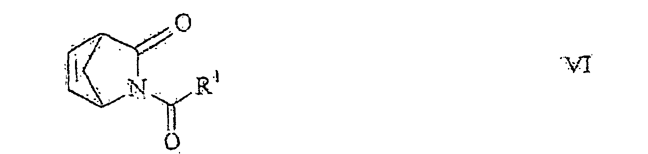 in which R¹ has the specified meaning, and in a second stage is reduced to the cyclopentene derivative having the general formula VII.

8. Method as claimed in claim 7, **characterised in that** the acylation in the first stage takes place with a carboxylic acid halogenide having the general formula 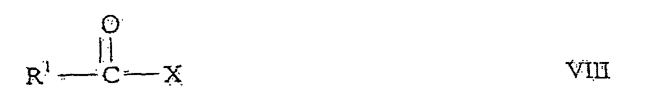 in which X stands for a halogen atom and R¹ has the specified meaning, or with a carboxylic acid anhydride having the general formula 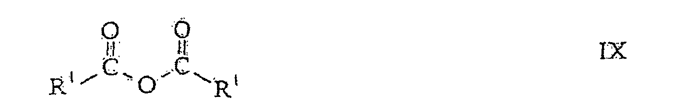 in which R¹ has the specified meaning.

9. Method as claimed in one of claims 7 or 8, **characterised in that** the acylation in the first stage takes place in an aprotic solvent.

10. Method as claimed in one of claims 7 to 9, **characterised in that** the reduction in the second stage takes place with an alkali or earth alkali metal boron hydride, an alkali or earth alkali metal aluminium hydride and/or with vitrid.

11. Method as claimed in one of claims 7 to 10, **characterised in that** the reduction in the second stage takes place in a protic solvent.

12. Method as claimed in one of claims 6 to 11, **characterised in that** the cyclopentene derivative having the general formula VII is reacted by means of microorganisms of the genus Rhodococcus, Gordona, Arthrobacter, Alcaligenes, Agrobacterium/Rhizobium, Bacillus, Pseudomonas or Alcaligenes/Bordetella.

13. Method as claimed in one of claims 6 to 12, **characterised in that** the cyclopentene derivative having the general formula VII is reacted by means of a Penicillin G acylase of microorganisms of the species Bacillus megaterium or Escherichia coli.

14. Method as claimed in one of claims 6 to 13, **characterised in that** in the third stage, the reaction is conducted at a temperature of 20 to 40°C and at a pH value of 5 to 9.

## Revendications

1. Microorganismes aptes à utiliser des dérivés de cyclopentène, choisis parmi les composés de formule générale 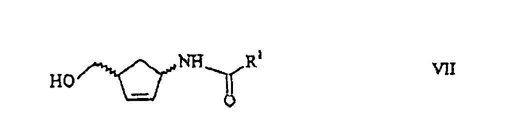 dans laquelle R¹ signifie un groupe alkyle en C₁ à C₄, alcoxy en C₁ à C₄, aryle ou aryloxy, comme unique source d'azote, comme unique source de carbone ou comme unique source de carbone et d'azote, **caractérisés en ce que** les microorganismes sont choisis parmi les espèces Alcaligenes / Bordetella FB 188 (DSM 1172), Rhodococcus erythropolis CB 101 (DSM 10686), Arthrobacter sp. HSZ 5 (DSM 10328), Rhodococcus sp. FB 387 (DSM 11291), Alcaligenes xylosoxydans ssp. denitrificans HSZ 17 (DSM 10329) ou Gordona sp. CB 100 (DSM 10687).

2. Enzyme présentant une activité de N-acétylaminoalcoolhydrolase susceptible d'être obtenue à partir de microorganismes qui sont aptes à utiliser au moins un dérivé de cyclopentène, choisi parmi les composés de formule générale 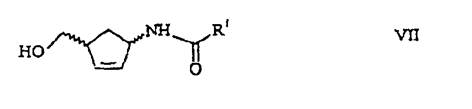 dans laquelle R¹ a la signification indiquée dans la revendication 1, comme unique source d'azote, comme unique source de carbone ou comme unique source de carbone et d'azote, l'enzyme étant apte à hydrolyser le composé de formule VII et
**caractérisée par**
a) une séquence d'acides aminés N-terminale Thr-Glu-Gln-Asn-Leu-His-Trp-Leu-Ser-Ala-Thr-Glu-Met-Ala-Ala-Ser-Val-Ala-Ser-Asn ; et
b) un poids moléculaire, déterminé par SDS-PAGE à 10-15%, de 50 kD.

3. Enzyme selon la revendication 2, en outre **caractérisée par**
c) un optimum de pH de 7,0 ± 1,0,
d) un optimum de température entre 25°C et 30°C à un pH de 7,0 ; et
e) une valeur de K_{M} pour le substrat N-acétyl-1-amino-4-hydroxyméthyl-2-cyclopentène de 22,5 mM ± 7,5 mM (30°C, 100 mM de tampon phosphate).

4. Enzyme selon la revendication 2 ou 3, susceptible d'être obtenue à partir de microorganismes du genre Rhodococcus, Gordona, Arthrobacter, Alcaligenes, Agrobacterium / Rhizobium, Bacillus, Pseudomonas ou Alcaligenes / Bordetella.

5. Enzyme selon l'une quelconque des revendications 2 à 4, susceptible d'être obtenue à partir de microorganismes choisis parmi les espèces Alcaligenes / Bordetella FB 188 (DSM 1172), Rhodococcus erythropolis CB 101 (DSM 10686), Arthrobacter sp. HSZ 5 (DSM 10328), Rhodococcus sp. FB 387 (DSM 11291), Alcaligenes xylosoxydans ssp. denitrificans HSZ 17 (DSM 10329) ou Gordona sp. CB 100 (DSM 10687).

6. Procédé pour la préparation de (1R,4S)-1-amino-4-(hydroxyméthyl)-2-cyclopentène ou (1S,4R)-1-amino-4-(hydroxyméthyl)-2-cyclopentène de formules I et II 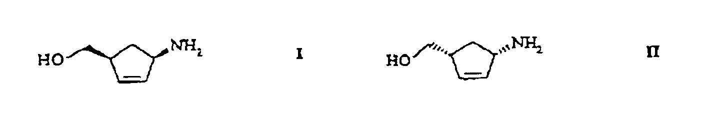 et/ou de dérivés de (1S,4R)-aminoalcool ou (1R,4S)-aminoalcool de formules générales III et IV 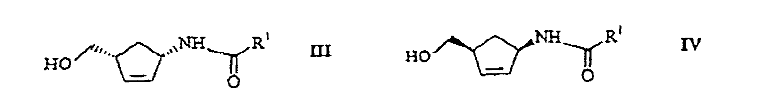 dans lesquelles R¹ signifie alkyle en C₁ à C₄, alcoxy en C₁ à C₄, aryle ou aryloxy, comprenant la transformation d'un dérivé de cyclopentène de formule générale 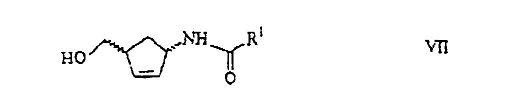 dans laquelle R¹ a la signification mentionnée, au moyen d'un microorganisme qui est apte à utiliser le composé de formule générale VII ci-dessus comme unique source d'azote, comme unique source de carbone ou comme unique source de carbone et d'azote, d'une enzyme présentant une activité d'acétylaminohydrolase susceptible d'être obtenue à partir de ces microorganismes et qui est apte à hydrolyser le composé selon la formule générale VII ci-dessus, et/ou d'une pénicilline G acylase en (1R,4S)-1-amino-4-(hydroxyméthyl)-2-cyclopentène ou (1S,4R)-1-amino-4-(hydroxyméthyl)-2-cyclopentène de formules I et II, ainsi que le cas échéant l'isolement de ces composés et/ou en dérivés (1S,4R)-aminoalcool ou (1R,4S)-aminoalcool des formules générales III et IV obtenus en plus du (1R,4S)-1-amino-4-(hydroxyméthyl)-2-cyclopentène ou (1S,4R)-1-amino-4-(hydroxyméthyl)-2-cyclopentène.

7. Procédé selon la revendication 6, **caractérisé en ce que** le dérivé de cyclopentène de formule générale 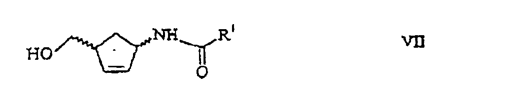 dans laquelle R¹ a la signification mentionnée est préparé **en ce que**, dans une première étape, on acyle la (±)-2-azabicyclo[2.2.1]hept-5-én-3-one de formule 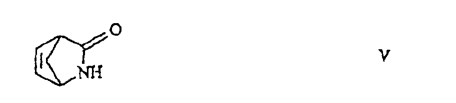 en un dérivé (±)-2-azabicyclo[2.2.1]hept-5-én-3-one de formule générale 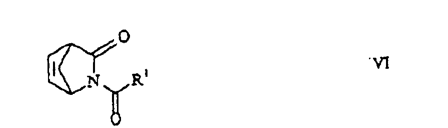 dans laquelle R¹ a la signification mentionnée, et on réduit ce composé dans une deuxième étape en dérivé de cyclopentène de formule générale VII.

8. Procédé selon la revendication 7, **caractérisé en ce qu'**on réalise l'acylation dans la première étape avec un halogénure d'acide carboxylique de formule générale 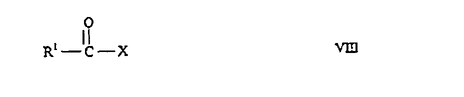 dans laquelle X représente un atome d'halogène et R¹ a la signification mentionnée, ou avec un anhydride d'acide carboxylique de formule générale 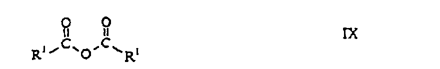 dans laquelle R' a la signification mentionnée.

9. Procédé selon l'une quelconque des revendications 7 ou 8, **caractérisé en ce qu'**on réalise l'acylation dans la première étape dans un solvant aprotique.

10. Procédé selon l'une quelconque des revendications 7 à 9, **caractérisé en ce qu'**on réalise la réduction dans la deuxième étape avec un borohydrure de métal alcalin ou alcalino-terreux, un aluminohydrure de métal alcalin ou alcalino-terreux et/ou du vitrure.

11. Procédé selon l'une quelconque des revendications 7 à 10, **caractérisé en ce qu'**on réalise la réduction dans la deuxième étape dans un solvant protique.

12. Procédé selon l'une quelconque des revendications 6 à 11, **caractérisé en ce que** la transformation du dérivé de cyclopentène de formule générale VII est réalisé au moyen de microorganismes du genre Rhodococcus, Gordona, Arthrobacter, Alcaligenes, Agrobacterium / Rhizobium, Bacillus, Pseudomonas ou Alcaligenes / Bordetella.

13. Procédé selon l'une quelconque des revendications 6 à 12, **caractérisé en ce que** la transformation du dérivé de cyclopentène de formule générale VII est réalisée au moyen d'une pénicilline G acylase de microorganismes de l'espèce Bacillus megaterium ou Escherichia coli.

14. Procédé selon l'une quelconque des revendications 6 à 13, **caractérisé en ce qu'**on réalise la transformation dans la troisième étape à une température de 20 à 40°C et à un pH de 5 à 9.


### SEQUENZPROTOKOLL

(1) ALLGEMEINE ANGABEN:.
   (i) ANMELDER:
      (A) NAME: LONZA AG
      (B) STRASSE: Muenchensteinerstrasse 38
      (C) ORT: Basel
      (E) LAND: Schweiz
      (F) POSTLEITZAHL: 4002
   (ii) BEZEICHNUNG DER ERFINDUNG: Verfahren zur Herstellung von einem Aminoalkohol
   (iii) ANZAHL DER SEQUENZEN: 1
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 20 Aminosäuren
      (B) ART: Aminosäure
      (C) STRANGFORM: nicht bekannt
      (D) TOPOLOGIE: nicht bekannt
   (ii) ART DES MOLEKÜLS: Protein
   (iii) HYPOTHETISCH: NEIN
   (iv) ANTISENSE: NEIN
   (v) ART DES FRAGMENTS: N-Terminus
   (vi) URSPRÜNGLICHE HERKUNFT:
      (B) STAMM: Rhodococcus erythropolis
      (C) INDIVIDUUM/ISOLAT: Rhodococcus erythropolis CB101
   (xi) SEQUENZBESCHREIBUNG: SEQ ID NO: 1:
   Thr Glu Gln Asn Leu His Trp Leu Ser Ala Thr Glu Met Ala Ala Ser
   1               5                   10                  15
   Val Ala Ser Asn
               20